# EUROPEAN PATENT APPLICATION

(11) **EP 0 815 813 A1**
(43) Date of publication of application: **07.01.1998**
(21) Application number: 97900459.5
(22) Date of filing: 20.01.1997
(51) Int. Cl.: A61F 5/56

(54) **CHIN FIXING APPARATUS**

(30) Priority: 19.01.1996 JP 24813/96
(71) Applicant: Keytron Co., Ltd., Taito-ku, Tokyo 111 (JP)
(72) Inventor: YOSHIDA, Norio, Ichikawa-shi, Chiba 272 (JP)
(74) Representative: Godwin, Edgar James
(86) International application number: JP9700104
(87) International publication number: WO9725948

(57) **Abstract**

A chin fixing apparatus rarely causing a user to have physical disorder during the use thereof and capable of reliably preventing the chin from moving back while the user sleeps. The chin fixing apparatus comprises a fixing piece and a filler. The fixing piece has an outer wall portion, an inner wall portion and a partition wall portion which are combined so as to have a substantially H-shaped cross section, whereby grooves for receiving parts of rows of teeth are formed on the upper and lower sides of the partition wall portion. Vertically extending through bores are formed in the partition wall portion. The filler can comprise a composition harmless to a human body and changing from the state of paste of a high viscosity into the state of gel having an elasticity in a short period of time. The fixing piece with the filler packed therein is bitten by the upper and lower teeth, which are bound together owing to the solidification of the filler, whereby the backward displacement of the chin is prevented.

## Description

### Technical Field

The present invention relates to a jaw fixing device capable of effectively preventing snore or apnea during sleeping.

### Background Art

When a person is asleep lying on his or her back, a lower jaw tends to recede as a result of relaxation of muscles in sleeping, so that a respiratory tract narrows at a pharynx. Therefore, one having such a tendency snores loudly as a uvula or its surrounding soil muscle vibrates, because the breathing air is forced to pass a narrow part of the pharynx during sleeping. In some cases, the pharynx is almost closed due to recession of the lower jaw, causing what is called apnea.

As an implement for preventing the lower jaw from receding during sleeping, there is known an implement to be placed in an oral cavity, which is called a sleep splint, such as a correcting implement disclosed in Japanese Examined Patent Publication No. 5-78346.

The implement disclosed in this publication is a sort of mouthpiece to be fitted on the upper row of teeth, and has a slanting portion extending downward from an upper portion of the implement for positioning the lower row of teeth more forward than the upper row of teeth, to thereby prevent the movement of the lower jaw and keep the respiratory tract at the pharynx wide open.

It has been confirmed that snore or apnea is considerably prevented by fitting this implement. However, since this type of conventional implement relies on its own force of clamping the teeth to fit it on a row of teeth, the clamping force is set strong to make the user feel uncomfortable while using the implement.

Further, the implement is fitted on a wide part of the upper row of teeth to constrict the upper row, and this also makes the user feel uncomfortable. In addition, the lower jaw is held in the forward position by the slanting surface insofar as the mouth remains closed, but there is no means to keep the user from opening his/her mouth unconsciously during sleeping. Thus, the user may become short of sleep because he/she feels uncomfortable with the implement fitted on the teeth. If the clamping force of the implement is set low, the implement may come off during sleeping. Further, the implement may not be effective since the user opens his/her mouth. For these reasons, the implement has not come into wide use.

The above deficiencies can be made up for to some extent by preparing the implement exactly fit to teeth of an individual user. However, making the implement in this manner necessitates the help of dentists and also daily attachment/detachment and maintenance of the implements are burdensome. This is also a cause of unpopularity of the implement.

### Disclosure of Invention

An object of the present invention is to provide a jaw fixing device which has a small size and can be used by anyone without reluctance, and also lessens the discomfort in use and can reliably prevent the lower jaw from receding during sleeping.

A jaw fixing device of the present invention comprises a fixing piece and a filler. The fixing piece is molded wiht rubber or synthetic resin harmless to the human organism. The fixing piece has outer and inner walls and a partition wall. The outer and inner walls extend in the vertical direction while the partition wall extends in the horizontal direction. The fixing piece has a section of substantially H-shape. Receiving grooves are defined above and below the partition wall, respectively, for individually receiving a part of a row of teeth, and through holes are formed in the vertical direction through the partition wall.

The filler is made of a composition which is harmless to the human organism and is capable of changing from a highly viscous paste state to an elastic gel state in a short time. For example, the filler may be a combination of a sol of silicone monomer and an agent for accelerating crosslinking in the air. Silicone polymer after hardening is not perfectly solid but a rather firm elastic material soil to the touch.

When using the jaw fixing device, a user fills the upper and lower receiving grooves of the fixing piece with the filler taken out of a container such as a tube, then bites the fixing piece with the upper and lower teeth, and waits for a while. When the fixing piece is bitten, the filler still has fluidity and therefore flows into the space between the receiving grooves of the fixing piece and the teeth and the gaps between the teeth, and further the through holes in the partition wall. The filler hardens there in a short time and thereby the teeth and the fixing piece, and thus the upper and lower teeth are connected to each other. Namely, the filler in the upper and lower receiving grooves joins together and hardens via the through holes of the partition wall, and the filler forced into the gaps between the teeth and hardened is present in the gap between bases of adjacent teeth like a wedge. I this state, the upper and lower teeth are no longer separable from each other, whereby the lower jaw is prevented from receding during sleeping

An adhesion of the filler to the teeth is achieved by a close contact, like a suction disk clinging to a surface of a wall, and also the hardened filler is not perfectly solid. Accordingly, when the jaw fixing device is not necessary any longer such as in the morning, it can be easily detached, for example, by deforming the fixing piece to allow the air to enter between the hardened filler and the teeth.

a distance between said outer and inner walls is set to be greater at a back teeth side than at a front teeth side in accordance with thickness of a part of the rows of teeth to be received.

The distance between the outer and inner walls is set to be greater at a back teeth side than at a front teeth side in accordance with thickness of a part of the rows of teeth to be received. With this arrangement, fittingness of the fixing piece is improved. The thickness of the partition wall is formed to be greater at the back teeth side than at the front teeth side. With this arrangement, forces of biting the fixing piece with the individual teeth is made even to enhance a stability of the fixing piece in use, and as a gap is formed between the upper and lower teeth, the user can breathe with ease while sleeping. The outer and inner walls are curved in accordance with the curvature of a part of the rows of teeth to be received, so that the fittingness is improved. Further, provision of a tab which protrudes forward from an outer surface of the outer wall facilitates a handling of the fixing piece at the time of attachment and detachment, and also eliminates a possibility of the fixing piece being swallowed by accident.

### Brief Description of Drawings

FIG. 1 is a perspective view showing an entire jaw fixing device according to an embodiment of the present invention;
FIG. 2a is a sectional view taken along a line II-II in FIG. 2b, FIGS. 2b is a plan view of a fixing piece of the jaw fixing device as shown in FIG. 1, FIG. 2c is a side view thereof;
FIG. 3 is a perspective view showing a fitting state of the jaw fixing device of FIG. 1 on rows of teeth;
FIG. 4 is a plan view showing fitting locations of the jaw fixing device of FIG. 1 on the lower jaw;
FIG. 5 is a perspective view showing a modification of a tab provided on the fixing piece; and
FIG. 6 is a perspective view showing a fitting state of a fixing piece with a tab having a hole for passing a string therethrough on rows of teeth.

### Best Mode of Carrying out the Invention

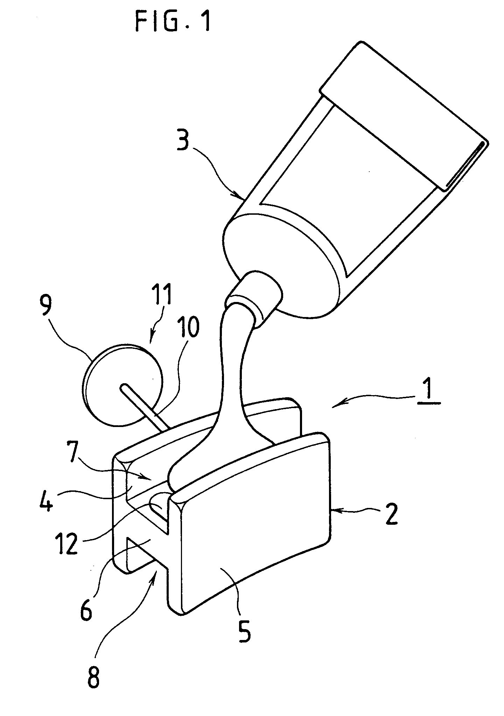

In FIG. 1, a jaw fixing device 1 comprises a fixing piece 2 and a filler 3. This jaw fixing device 1 is of a disposable type which is discarded after a single usage.

The fixing piece 2 is molded with a natural rubber or a synthetic resin such as silicone rubber which are harmless to the human organism, and has an outer wall 4, an inner wall 5 and a partition wall 6. The outer and inner walls 4 and 5 are arranged substantially parallel to each other in a vertical direction, and the partition wall 6 extends in a horizontal direction to connect the outer and inner walls 4 and 5. Accordingly, the fixing piece 2 has a substantially H-shaped section and receiving grooves 7 and 8 are defined above and below the partition wall 6 for respectively receiving a part of a row of teeth. In this embodiment, the part of a row of teeth means three to four teeth arranged in a row. Ridges and corners of the outer wall 4, the inner wall 5 and the partition wall 6 are rounded off.

A tab 11, which comprises a thin rod-like member 10 with a small disk 9 at its distal end, protrudes from a front surface of the outer wall 4. The partition wall 6 has a plurality of through holes 12 formed therein in the vertical direction.

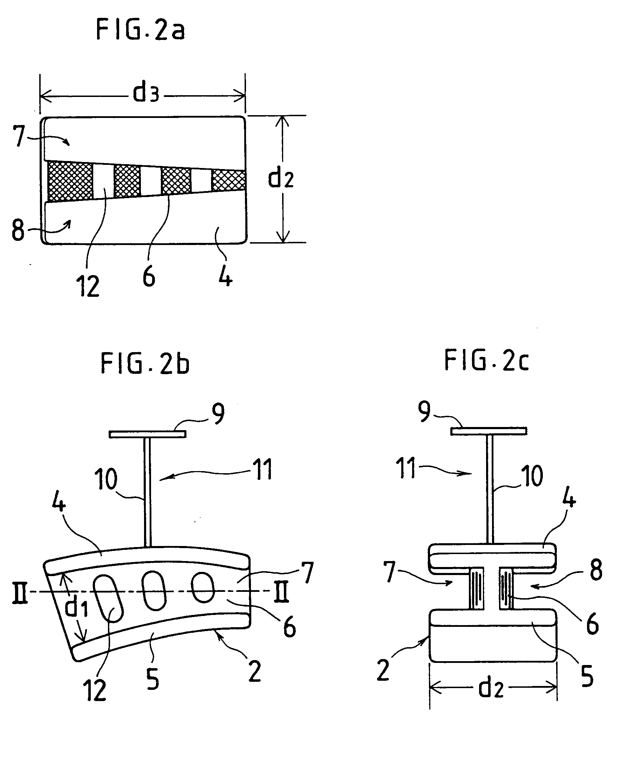

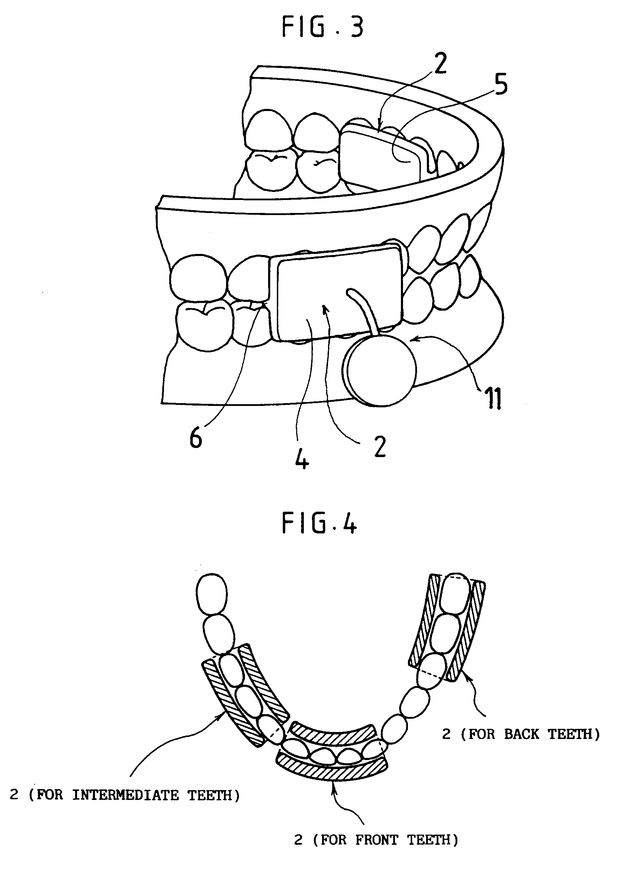

As shown in FIGS. 2a-2c, the outer and inner walls 4 and 5, each having a thickness of about 1 to 3 mm, are arranged substantially parallel to each other and, in this embodiment, are curved in accordance with an arc of the part of the rows of teeth to be received, as viewed in plan. The distance d₁ between the outer and inner walls 4 and 5 is set in accordance with the thicknesses of the rows of teeth to be received in such a manner that the distance at one end of the fixing piece closer to back teeth is greater than that at the other end of the fixing piece closer to the front teeth. The height d₂ of the outer and inner walls 4 and 5 is nearly equal to a distance between upper and lower gums when the upper and lower teeth are occluded, and is approximately 24 mm (see FIG. 3). The width in the lateral direction d₃ of the outer and inner walls is set to a value slightly greater than the distance between opposite ends of the part of the rows of teeth to be received, and is approximately 25 to 30 mm, for example.

The thickness of the partition wall 6 at one end thereof closer to the back teeth to be received is greater than that at the other end closer to the front teeth, and a plurality of through holes 12 are provided vertically through the partition wall. The thickness of the partition wall 6 is varied so as to conform with the gap naturally formed when the upper and lower teeth are occluded, but it is not essential to give the partition wall the varying thickness.

The distance d₁ between the outer and inner walls 4 and 5, the degree of their curvature, and the thickness of the partition wall 6 are set in accordance with the location of the rows of teeth where the fixing piece 2 is to be fitted, i.e., front teeth, intermediate teeth, or back teeth, as shown in FIG. 4.

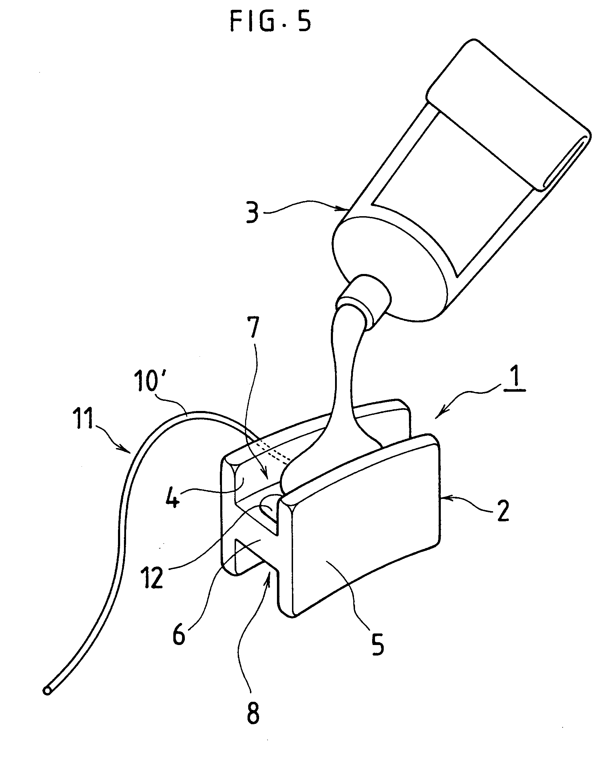

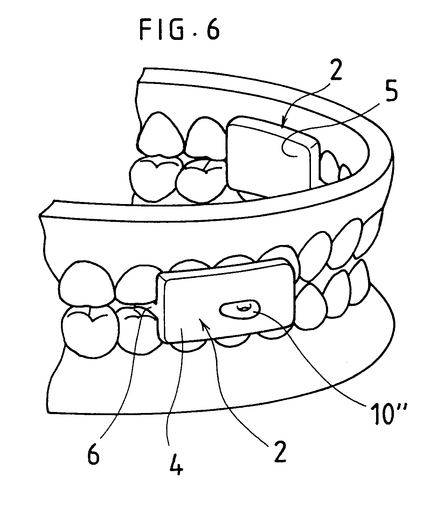

The tab 11 is provided so as to prevent a user from swallowing the fixing piece 2, and its shape may be defined relatively freely insofar as the tab interferes as little as possible when a mouth is closed with the fixing piece 2 fitted. For example, as shown in FIG. 5, the tab 11 may be a string 10' having one end embedded in the outer wall 4 and extending from a central part of the front surface of the outer wall 4. Further, as shown in FIG. 6, the tab may be a protuberance 10'' having a hole through which a string can be passed and tied.

The filler is what is called an RTV (room-thermovulcanizer) which is a pasty mixture of silicone monomer and an agent for accelerating crosslinking in the air. As such a material, a dental modeling agent for modeling teeth or a commercially available agent for gumming artificial teeth (a pasty material which contains sodium carboxymethylcellulose or polyethylene oxide as a main component and gels when absorbing water) can be used. In this embodiment, a quantity of filler necessary for a single usage is contained in a squeeze tube to be used in combination with the fixing piece. Alternatively, a pasty base material may be mixed with a catalyst for crosslinking. In this case, the pasty base material and the catalyst are contained in separate containers, and the necessary quantities of the base material and catalyst for a single usage are taken out of the respective containers and mixed together for use.

The jaw fixing device 1 is used in the manner described below.

The fixing piece 2 and the filler 3 are prepared before sleeping and the tube is squeezed to fill the upper and lower receiving grooves 7 and 8 of the fixing piece 2 with the filler.

The user places the fixing piece 2 between the user's upper and lower teeth holding the outer wall 4 and clenches the teeth (FIG. 3), to wait until the filler hardens (two to three minutes).

When the teeth are clenched, the filler 3 fills a space between the receiving grooves 7, 8 of the fixing piece 2 and the teeth, gaps between the teeth and further the through holes 12 in the partition wall 6, to harden closely contacting with surfaces of the teeth and surfaces of the individual walls. In this state, the filler 3 in the upper and lower receiving grooves 7 and 8 joins together via the through holes 12, and enters into the gaps between the teeth in rows in the form of wedge. After the filler 3 hardens, it serves as an adhesive for fixing the fixing piece 2 to the teeth, thus making it difficult for the user to open his/her mouth involuntarily.

Since the fixing piece 2 has the partition wall 6, a gap is formed between the upper and lower rows of teeth even though the mouth can not be opened, so that the user can breathe without difficulty while sleeping. The filler 3 hardens only to such an extent that its hardness is equivalent to that of a firm elastic material, and therefore a tongue or lips are never injured even when they touch the hardened filler 3.

The space d₁ between the outer and inner walls 4 and 5 of the fixing piece 2 and degrees of curvature thereof are varied in accordance with a position where the fixing piece 2 is designed to be fitted to the rows of teeth (FIG. 4), and therefore the fixing piece 2 needs to be fitted in proper position according to a selected type (for front teeth, back teeth, or intermediate teeth).

The fitting position is selected so that the fixing piece can be easily handled and the user feels less uncomfortable, and a treated part such as a post crown or a bridge should be avoided if possible.

Once the teeth of the lower jaw are fixed to those of the upper jaw in the above-mentioned manner, the lower jaw never recedes and thus the respiratory tract at the pharynx is not narrowed even when the muscles are relaxed during sleep. It is therefore possible to prevent snore or apnea during sleep.

After the user awakes in the morning, the fixing piece 2 is no longer necessary, and the user pulls off the fixing piece 2 from the rows of teeth with his/her fingers hooked on the upper and lower edges of the outer wall 4. At this time, since the filler 3 is merely in close contact with the teeth and is not chemically bonded to the teeth, the fixing piece 2 can be easily released from the close contact and thus detached. Although the fixing piece 2 thus detached has the filler 3 adhering thereto, the adhesion of the hardened filler 3 to the fixing piece 2 does not raise a problem because the fixing piece 2 is of a disposable type in principle.

The jaw fixing device may alternatively be designed for repeated use. However, as a hygienic maintenance by washing, for example, is burdensome and in view of safety, the jaw fixing device is preferably of a disposable type.

Further, the distance d₁ between the outer and inner walls 4 and 5 of the fixing piece 2 and the degree of curvature thereof need not be set to exactly fit the rows of teeth of individual users. The fixing piece 2 is rather made somewhat large to effectively use the filler 3 and to absorb dimensional differences among individual users.

According to the present invention, snore or apnea during sleep can be reliably prevented by a device of a simple structure. The outer and inner walls and the partition wall do not clamp the teeth for fixing the upper and lower teeth to each other by the clamping force, so that the walls can be reduced in thickness and the fixing piece can be reduced in weight. Therefore, it is possible to make the user feel less uncomfortable when he/she is wearing the fixing piece in his/her mouth. It is also possible to improve the fittingness of the jaw fixing device, thereby further lessening the discomfort caused by the device in the mouth. Furthermore, the partition wall can be bitten evenly with the upper and lower teeth to be fixed to each other, so that the user feels the jaw fixing device to be stabilized while using the device. The fixing piece is fitted on a part of the rows of teeth and thus there is formed a gap between the rows of teeth where the fixing piece is not fitted, whereby the user is allowed to breathe with ease while sleeping. Also, since the outer and inner walls are formed so as to extend along the arcs of the rows of teeth, the fixing piece fits the rows of teeth well, making it possible to lessen a discomfort caused by the fixing piece in the mouth. Further, it is easy to handle the fixing piece at the time of attachment and detachment, and also the possibility that the fixing piece is swallowed by accident can be eliminated.

## Claims

1. A jaw fixing device comprising:
a fixing piece made of rubber or synthetic resin harmless to human organism and having a section of substantially H-shape, said fixing piece having outer and inner walls extending in a vertical direction, a partition wall extending in a horizontal direction, receiving grooves defined above and below said partition wall, respectively, for individually receiving therein a part of a row of teeth, and a through hole extending in the vertical direction through said partition wall; and
a filler made of a composition harmless to human organism, the composition being changeable from a highly viscous paste state to an elastic gel state in a short time.

2. A jaw fixing device according to claim 1, wherein a distance between said outer and inner walls is set to be greater at a back teeth side than at a front teeth side in accordance with thickness of a part of the rows of teeth to be received.

3. A jaw fixing device according to claim 2, wherein said partition wall is formed so that thickness thereof at the back teeth side is greater than that at the front teeth side.

4. A jaw fixing device according to claim 2, wherein said outer and inner walls are curved in accordance with curvature of a part of the rows of teeth to be received.

5. A jaw fixing device according to claim 1, wherein said partition wall is formed so that thickness thereof at the back teeth side is greater than that at the front teeth side.

6. A jaw fixing device according to claim 5, wherein said outer and inner walls are curved in accordance with curvature of a part of the rows of teeth to be received.

7. A jaw fixing device according to claim 1, wherein said outer and inner walls are curved in accordance with curvature of a part of the rows of teeth to be received.

8. A jaw fixing device according to any one of claims 1 through 7, further comprising a tab protruding from an outer surface of said outer wall.

9. A jaw fixing device according to claim 8, wherein said tab comprises a thin rod-like member having a small disk at a distal end thereof.

10. A jaw fixing device according to claim 8, wherein said tab comprises a string protruding from the outer surface of said outer wall.

11. A jaw fixing device according to claim 8, wherein said tab comprises a protuberance having a hole for passing a string therethrough.
